(19) Europäisches Patentamt
European Patent Office
Office européen des brevets

(11) EP 1 011 619 B2

(12) **NOUVEAU FASCICULE DE BREVET EUROPEEN**
Après la procédure d'opposition

(45) Date de publication et mention de la décision concernant l'opposition:
**04.07.2012 Bulletin 2012/27**

(45) Mention de la délivrance du brevet:
**14.09.2005 Bulletin 2005/37**

(21) Numéro de dépôt: **98939723.7**

(22) Date de dépôt: **20.07.1998**

(51) Int Cl.:
***A61K 8/49*** *(2006.01)* ***A61Q 5/10*** *(2006.01)*

(86) Numéro de dépôt international:
**PCT/FR1998/001591**

(87) Numéro de publication internationale:
**WO 1999/011231 (11.03.1999 Gazette 1999/10)**

# (54) COMPOSITION POUR LA TEINTURE D'OXYDATION DES FIBRES KERATINIQUES

ZUSAMMENSETZUNG FÜR DIE OXIDATIONSFÄRBUNG VON KERATINISCHEN FASERN

DYEING COMPOSITION FOR KERATIN FIBRES

(84) Etats contractants désignés:
**AT BE CH CY DE DK ES FI FR GB GR IE IT LI LU MC NL PT SE**

(30) Priorité: **01.09.1997 FR 9710857**

(43) Date de publication de la demande:
**28.06.2000 Bulletin 2000/26**

(73) Titulaire: **L'Oréal**
**75008 Paris (FR)**

(72) Inventeurs:
- **MAUBRU, Mireille**
  **F-78400 Chatou (FR)**
- **AUDOUSSET, Marie-Pascale**
  **F-92600 Asnières (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:

| | |
|---|---|
| **EP-A- 0 728 466** | **EP-A- 0 740 931** |
| **EP-A1- 0 375 977** | **WO-A1-96/15766** |
| **DE-A- 19 543 988** | **DE-A1- 4 234 887** |

EP 1 011 619 B2

## Description

La présente invention a pour objet une composition pour la teinture d'oxydation des fibres kératiniques, en particulier des fibres kératiniques humaines telles que les cheveux, comprenant au moins une base d'oxydation choisie parmi les diamino pyrazoles, en association avec au moins un méta-aminophénol halogéné en ortho du phénol à titre de coupleur, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être choisis notamment parmi des métadiamines aromatiques, des métaaminophénols, des métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demandes de brevet allemand DE 3 843 892, DE 4 234 887, DE 4 234 886, DE 4 234 885 ou DE 195 43 988 des compositions pour la teinture d'oxydation des fibres kératiniques contenant à titre de base d'oxydation des dérivés de pyrazole tels que des 4,5-diamino pyrazoles, des 3,4-diamino pyrazoles ou des 3,4,5-triamino pyrazoles, en association avec des coupleurs classiquement utilisés pour la teinture d'oxydation, tels que par exemple des métaphénylènediamines, des méta-aminophénols, des métadiphénols et des coupleurs hétérocycliques tels que par exemple des dérivés indoliques. De telles compositions ne sont cependant pas entièrement satisfaisantes notamment du point de vue de la tenue des colorations obtenues vis à vis des diverses agressions que peuvent subir les cheveux et en particulier vis à vis de la transpiration.

Or, la demanderesse vient maintenant de découvrir qu'il est possible d'obtenir de nouvelles teintures puissantes et particulièrement résistantes aux diverses agressions que peuvent subir les cheveux, en associant, à titre de base d'oxydation, au moins un diamino pyrazole et à titre de coupleur un méta-aminophénol halogéné en position ortho du phénol.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation choisie parmi les diamino pyrazoles ;
- et au moins un coupleur choisi parmi les méta-aminophénols halogénés de formule (I) suivante, et leurs sels d'addition avec un acide :

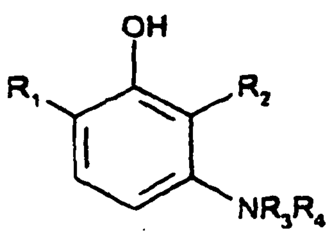

(I)

dans laquelle :

- $R_1$ et $R_2$, qui peuvent être identiques ou différents, représentent un atome d'hydrogène, un atome d'halogène tel que le chlore, le brome, l'iode ou le fluor, un radical alkyle en $C_1$-$C_4$, monohydroxyalkyle en $C_1$-$C_4$, polyhydroxyalkyle en $C_2$-$C_4$, alcoxy en $C_1$-$C_4$, monohydroxyalcoxy en $C_1$-$C_4$ ou polyhydroxyalcoxy en $C_2$-$C_4$ ;
- $R_3$ et $R_4$, qui peuvent être identiques ou différents, représentent un atome d'hydrogène, un radical alkyle en $C_1$-$C_4$, monohydroxyalkyle en $C_1$-$C_4$, polyhydroxyalkyle en $C_2$-$C_4$ ou monoaminoalkyle en $C_1$-$C_4$ ;

étant entendu qu'au moins un des radicaux $R_1$ et $R_2$ représente un atome d'halogène.

La composition de teinture d'oxydation conforme à l'invention permet d'obtenir des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables notamment en ce qui concerne la résistance des colorations vis à vis de la transpiration.

Parmi les radicaux alkyle en $C_1$-$C_4$ et alcoxy en $C_1$-$C_4$ des composés de formule (I) ci-dessus, on peut citer notamment les radicaux méthyle, éthyle, propyle, méthoxy et éthoxy.

Parmi les méta-aminophénols halogénés de formule (I), on peut plus particulièrement citer le 3-amino 6-chloro phénol, le 3-amino 6-bromo phénol, le 3-(β-aminoéthyl)amino 6-chloro phénol, le 3-(β-hydroxyéthyl)amino 6-chloro phénol, le 3-amino 2-chloro 6-méthyl phénol, et leurs sels d'addition avec un acide.

Parmi les diamino pyrazoles utilisables à titre de base d'oxydation dans les compositions tinctoriales conformes à l'invention, on peut plus particulièrement citer :

a) les diamino pyrazoles de formule (II) suivante et leurs sels d'addition avec un acide :

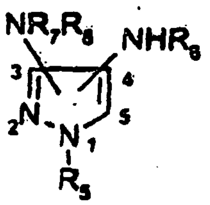

(II)

dans laquelle :

- $R_5$ représente un atome d'hydrogène, un radical alkyle en $C_1$-$C_6$, hydroxyalkyle en $C_2$-$C_4$, benzyle, phényle, benzyle substitué par un atome d'halogène ou par un groupe alkyle en $C_1$-$C_4$, ou alcoxy en $C_1$-$C_4$, ou forme avec l'atome d'azote du groupement $NR_7R_8$ en position 5 un hétérocycle héxahydropyridazinique ou tétrahydropyrazolique éventuellement monosubstitué par un groupe alkyle en $C_1$-$C_4$ ;
- $R_6$ et $R_7$, identiques ou différents, représentent un atome d'hydrogène, un radical alkyle en $C_1$-$C_4$, hydroxyalkyle en $C_2$-$C_4$, benzyle ou phényle ;
- $R_8$ représente un atome d'hydrogène, un radical alkyle en $C_1$-$C_6$ ou hydroxyalkyle en $C_2$-$C_4$ ; sous réserve que $R_6$ représente un atome d'hydrogène lorsque $R_5$ représente un radical benzyle substitué ou forme un hétérocycle avec l'atome d'azote du groupement $NR_7R_8$ en position 5 ;

b) les diamino pyrazoles de formule (III) suivante, et leurs sels d'addition avec un acide :

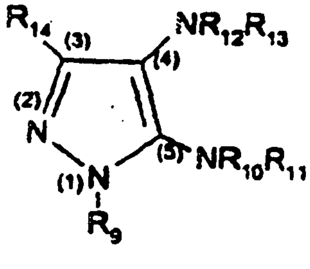

(III)

dans laquelle :

- $R_9$, $R_{10}$, $R_{11}$, $R_{12}$ et $R_{13}$, identiques ou différents, représentent un atome d'hydrogène ; un radical alkyle en $C_1$-$C_6$ linéaire ou ramifié ; un radical hydroxyalkyle en $C_2$-$C_4$ ; un radical aminoalkyle en $C_2$-$C_4$ ; un radical phényle ; un radical phényle substitué par un atome d'halogène ou un radical alkyle en $C_1$-$C_4$, alcoxy en $C_1$-$C_4$, nitro, trifluorométhyle, amino ou alkylamino en $C_1$-$C_4$ ; un radical benzyle ; un radical benzyle substitué par un atome d'halogène ou par un radical alkyle en $C_1$-$C_4$, alcoxy en $C_1$-$C_4$, méthylènedioxy ou amino ; ou un radical

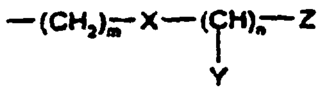

dans lequel m et n sont des nombres entiers, identiques ou différents, compris entre 1 et 3 inclusivement, X représente un atome d'oxygène ou bien le groupement NH, Y représente un atome d'hydrogène ou bien un radical méthyle, et Z représente un radical méthyle, un groupement OR ou NRR' dans lesquels R et R', qui peuvent être identiques ou différent, désignent un atome d'hydrogène, un radical méthyle ou un radical éthyle, étant entendu que lorsque $R_{10}$ représente un atome d'hydrogène, alors $R_{11}$ peut également représenter un radical amino ou alkylamino en $C_1$-$C_4$,
- $R_{14}$ représente un radical alkyle en $C_1$-$C_6$, linéaire ou ramifié ; un radical hydroxyalkyle en $C_1$-$C_4$, ; un radical aminoalkyle en $C_1$-$C_4$ ; un radical alkyle ($C_1$-$C_4$)aminoalkyle en $C_1$-$C_4$ ; un radical dialkyle ($C_1$-$C_4$)-aminoalkyle en $C_1$-$C_4$ ; un radical hydroxyalkyle ($C_1$-$C_4$)-amino alkyle en $C_1$-$C_4$ ; un radical alcoxy ($C_1$-$C_4$) méthyle ; un radical phényle ; un radical phényle substitué par un atome d'halogène ou par un radical alkyle en $C_1$-$C_4$, alcoxy en $C_1$-$C_4$, nitro, trifluorométhyle, amino ou alkylamino en $C_1$-$C_4$ ; un radical benzyle ; un radical benzyle substitué par un atome d'halogène ou par un radical alkyle en $C_1$-$C_4$, alcoxy en $C_1$-$C_4$, nitro, trifluorométhyle, amino ou alkylamino en $C_1$-$C_4$ ; un hétérocycle choisi parmi le thiophène, le furane et la pyridine, ou encore un radical $-(CH_2)_p-O-(CH_2)_q-OR''$, dans lequel p et q sont des nombres entiers, identiques ou différents, compris entre 1 et 3 inclusivement et R'' représente un atome d'hydrogène ou un radical méthyle,

étant entendu que dans la formule (III) ci-dessus :

- au moins un des radicaux $R_{10}$, $R_{11}$, $R_{12}$ et $R_{13}$ représente un atome d'hydrogène,
- lorsque que $R_{10}$, respectivement $R_{12}$, représente un radical phényle substitué ou non, ou un radical benzyle ou un radical

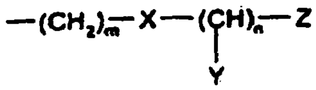,

alors $R_{11}$, respectivement $R_{13}$, ne peut représenter aucun de ces trois radicaux,
- lorsque $R_{12}$ et $R_{13}$ représentent simultanément un atome d'hydrogène, alors $R_9$ peut former, avec $R_{10}$ et $R_{11}$, un hétérocycle hexahydropyrimidinique ou tétrahydroimidazolique éventuellement substitué par un radical alkyle en $C_1$-$C_4$

ou 1,2,4-tétrazolique,

- lorsque $R_{10}$, $R_{11}$, $R_{12}$ et $R_{13}$ représentent un atome d'hydrogène ou un radical alkyle en $C_1$-$C_6$, alors $R_9$ ou $R_{14}$ peut également représenter un reste hétérocyclique 2, 3 ou 4-pyridyle, 2 ou 3-thiényle, 2 ou 3-furyle éventuellement substitué par un radical méthyle ou bien encore un radical cyclohexyle.

Parmi les diamino pyrazoles de formule (II) ci-dessus, on peut plus particulièrement citer le 4,5-diamino 1-(4'-méthoxybenzyle) pyrazole, le 4,5-diamino 1-(4'-méthylbenzyle) pyrazole, le 4,5-diamino 1-(4'-chlorobenzyle) pyrazole, le 4,5-diamino 1-(3'-méthoxybenzyle) pyrazole, le 4-amino 1-(4'-méthoxybenzyle) 5-méthylamino pyrazole, le 4-amino 5-(β-hydroxyéthyl)amino 1-(4'-méthoxybenzyle) pyrazole, le 4-amino 5-(β-hydroxyéthyl)amino 1-méthyl pyrazole, le 4-amino (3) 5-méthylamino pyrazole, le 3-(5),4-diamino pyrazole, le 4,5-diamino 1-méthyl pyrazole, le 4,5-diamino 1-benzyl pyrazole, la 3-amino 4,5,7,8-tétrahydro pyrazolo [1,5-a] pyrimidine, le 7-amino 2,3-dihydro 1-H-imidazol [1,2-b] pyrazole, la 3-amino 8-méthyl 4,5,7,8-tétrahydropyrazolo [1,5-a] pyrimidine, et leurs sels d'addition avec un acide.

Les diamino pyrazoles de formule (III) sont des composés connus qui peuvent être préparés selon le procédé de synthèse tel que décrit par exemple dans la demande de brevet français FR-A-2 733 749.

Parmi les diamino pyrazoles de formule (III) ci-dessus, on peut plus particulièrement citer :

- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino 1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino 3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphényl) pyrazole,
- le 4,5-diamino 3-méthyl 1-(3'-méthoxyphényl) pyrazole,
- le 4,5-diamino 3-méthyl 1-(4'-méthoxyphényl) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminàméthyl 1-méthyl pyrazole,
- le 4,5-diamino 3-diméthylaminométhyl 1-éthyl pyrazole,
- le 4,5-diamino 3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,
- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phènyl 3-méthyl pyrazole,
- le 4-amino 5-(β-hydroxyéthyl)amino 1-(2-méthoxyphényl) 3-méthyl pyrazole,
- le 4-amino 5-(β-hydroxyéthyt)amino 1-(3-méthoxyphényl) 3-méthyl pyrazole,
- le 4-amino 5-(β-hydroxyéthyl)amino 1-(4-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 4,5-diamino 1,3-diméthyl pyrazole,

- le 4,5-diamino 3-tert.butyl 1-méthyl pyrazole,
- le 4,5-diamino 1-tert.butyl 3-méthyl pyrazole,
- le 4,5-diamino 1-méthyl 3-phényl pyrazole,
- le 4,5-diamino 1-(β-hydroxyéthyl) 3-méthyl pyrazole,
- le 4,5-diamino 1-(β-hydroxyéthyl) 3-phényl pyrazole,
- le 4,5-diamino 1-méthyl 3-(2'-chlorophényl) pyrazole,
- le 4,5-diamino 1-méthyl 3-(4'-chlorophényl) pyrazole,
- le 4,5-diamino 1-méthyl 3-(3'-trifluorométhylphényl) pyrazole,
- le 4,5-diamino 1,3-diphényl pyrazole,
- le 4,5-diamino 3-méthyl 1-phényl pyrazole,
- le 4-amino 1,3-diméthyl 5-phénylamino pyrazole,
- le 4-amino 1-éthyl 3-méthyl 5-phénylamino pyrazole,
- le 4-amino 1,3-diméthyl 5-méthylamino pyrazole,
- le 4-amino 3-méthyl 1-isopropyl 5-méthylamino pyrazole,
- le 4-amino 3-isobutoxyméthyl 1-méthyl 5-méthylamino pyrazole,
- le 4-amino 3-méthoxyéthoxyméthyl 1-méthyl 5-méthylamino pyrazole,
- le 4-amino 3-hydroxyméthyl 1-méthyl 5-méthylamino pyrazole,
- le 4-amino 1,3-diphényl 5-phénylamino pyrazole,
- le 4-amino 3-méthyl 5-méthylamino 1-phényl pyrazole,
- le 4-amino 1,3-diméthyl 5-hydrazino pyrazole,
- le 5-amino 3-méthyl 4-méthylamino 1-phényl pyrazole,
- le 5-amino 1-méthyl 4-(N,N-méthylphényl)amino 3-(4'-chlorophényl) pyrazole,
- le 5-amino 3-éthyl 1-méthyl 4-(N,N-méthylphényl)amino pyrazole,
- le 5-amino 1-méthyl 4-(N,N-méthylphényi)amino 3-phényl pyrazole,
- le 5-amino 3-éthyl 4-(N,N-méthylphényl)amino pyrazole,
- le 5-amino 4-(N,N-méthylphényl)amino 3-phényl pyrazole,
- le 5-amino 4-(N,N-méthylphényl)amino 3-(4'-méthylphényl) pyrazole,
- le 5-amino 3-(4'-chlorophényl) 4-(N,N-méthylphényl)amino pyrazole,
- le 5-amino 3-(4'-mèthoxyphényl) 4-(N,N-méthylphényl)amino pyrazole,
- le 4-amino 5-méthylamino 3-phényl pyrazole,
- le 4-amino 5-éthylamino 3-phényl pyrazole,
- le 4-amino 5-éthylamino 3-(4'-méthylphényl) pyrazole,
- le 4-amino 3-phényl 5-propylamino pyrazole,
- le 4-amino 5-butylamino 3-phényl pyrazole,
- le 4-amino 3-phényl 5-phénylamino pyrazole,
- le 4-amino 5-benzylamino 3-phényl pyrazole,
- le 4-amino 5-(4'-chlorophényl)amino 3-phényl pyrazole,
- le 4-amino 3-(4'-chtorophényl) 5-phénylamino pyrazole,
- le 4-amino 3-(4'-méthoxyphényl) 5-phénylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoèthyl)amino 1,3-diméthyl pyrazole,

et leurs sels d'addition avec un acide.

Parmi ces diamino pyrazoles de formule (III) ci-dessus, on préfère plus particulièrement :

- le 4,5-diamino 1,3-diméthyl pyrazole,
- le 4,5-diamino 3-méthyl 1-phényl pyrazole,
- le 4,5-diamino 1-méthyl 3-phényl pyrazole,
- le 4-amino 1,3-diméthyl 5-hydrazino pyrazole,
- le 1-benzyl 4,5-diamino 3-méthyl pyrazole,
- le 4,5-diamino 3-tert-butyl 1-méthyl pyrazole,
- le 4,5-diamino 1-tert-butyl 3-méthyl pyrazole,
- le 4,5-diamino 1-(β-hydroxyéthyl) 3-méthyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 1-éthyl 3-hydroxyméthyl pyrazole,

- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,

et leurs sels d'addition avec un acide.

Le ou les diamino pyrazoles conformes à l'invention et/ou le ou les sels d'addition avec un acide correspondants représentent de préférence de 0,0005 à 12 % en poids environ du poids total de la composition tinctoriale, et encore plus préférentiellement de 0,005 à 6 % en poids environ de ce poids.

Le ou les méta-aminophénols halogénés de formule (I) conformes à l'invention et/ou le ou les sels d'addition avec un acide correspondants, représentent de préférence de 0,0001 à 5 % en poids environ du poids total de la composition tinctoriale, et encore plus préférentiellement de 0,005 à 3 % en poids environ de ce poids.

Les compositions tinctoriales conformes à l'invention peuvent contenir d'autres coupleurs classiquement utilisés pour la teinture d'oxydation, différents des méta-aminophénols halogénés de formule (I), et/ou d'autres bases d'oxydation classiquement utilisées pour la teinture d'oxydation, différentes d'un diamino pyrazole et/ou des colorants directs notamment pour modifier les nuances ou les enrichir en reflets.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyèthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante :

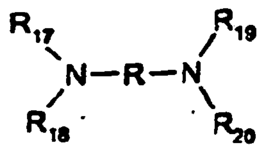

(V)

dans laquelle R est un reste propylène éventuellement substitué par un groupement hydroxyle ou un radical alkyle en $C_1$-$C_4$ ; $R_{17}$, $R_{18}$, $R_{19}$ et $R_{20}$, identiques ou différents, représentent un atome d'hydrogène, un radical alkyle en $C_1$-$C_4$ ou hydroxyalkyle en $C_1$-$C_4$.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas,

ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 60 minutes environ, de préférence 5 à 40 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates, percarbonates et persulfates, les peracides. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

## EXEMPLES

## <u>EXEMPLES DE TEINTURE COMPARATIFS 1 à 4</u>

On a préparé les compositions tinctoriales, conformes à l'invention, suivantes (teneurs en grammes) :

| EXEMPLE | 1 (*) | 2 | 3 | 4 |
|---|---|---|---|---|
| 4,5-diamino 1-éthyl 3-méthyl pyrazole, 2 HCl (base d'oxydation) | 0,639 | 0,639 | 0,639 | 0,639 |
| 3-amino phénol. (coupleur ne faisant pas partie de l'invention) | 0,327 | - | - | - |
| 3-amino 6-choro phénol (coupleur conforme à l'invention) | - | 0,431 | - | - |
| 3-(β-aminoéthyl)amino 6-chloro phénol (coupleur conforme à l'invention) | | - | 0,560 | - |
| 3-(β-hydroxyéthyl)amino 6-chloro phénol (coupleur conforme à l'invention) | - | - | - | 0,563 |
| Support de teinture commun | (**) | (**) | (**) | (**) |

(suite)

| EXEMPLE | 1 (*) | 2 | 3 | 4 |
|---|---|---|---|---|
| Eau déminéralisée q.s.p. | 100g | 100 g | 100 g | 100 g |

(*) : exemple ne faisant pas partie de l'invention
(**) support de teinture commun :
- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g
- Amine oléique à 2 moles d'oxyde d'éthylène vendue sous la dénomination commercial ETHOMEEN O12 ® par la société AKZO 7,0 g
- Laurylamino succinamate de diéthylaminopropyle, sel de sodium, à 55% de M.A. 3,0 g M.A.
- Alcool oléique 5,0 g
- Diéthanolamide d'acide oléique 12,0 g
- Propylèneglycol 3.5 g
- Alcool éthylique 7,0 g
- Dipropylèneglycol 0,5 g
- Monométhyléther de propylèneglycol 9,0 g
- Métabisulfite de sodium en solution aqueuse, à 35 % de M.A. 0,455 g M.A.
- Acétate d'ammonium 0.8 g
- Antioxydant, séquestrant q.s.
- Parfum, conservateur q.s.
-Ammoniaque à 20% de $NH_3$ 10 g

Il est important de noter que chacune des compositions tinctoriales 1 à 4 ci-dessus contient la même quantité molaire de coupleur, à savoir $3.10^{-3}$ mole.

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes et ont ensuite été soumises à un test de résistance à l'action de la transpiration.

La couleur des mèches de cheveux teintes avec les compositions 1 à 4 a été évaluée dans le système MUNSELL au moyen d'un colorimètre CM 2002 MINOLTA ®, avant le test de résistance à l'action de la transpiration.

Selon la notation MUNSELL, une couleur est définie par l'expression H V / C dans laquelle les trois paramètres désignent respectivement la teinte ou Hue (H), l'intensité ou Value (V) et la pureté ou Chromaticité (C), la barre oblique de cette expression est simplement une convention et n'indique pas un ratio.

Les mèches de cheveux teintes ont ensuite été soumises au test de résistance à l'action de la transpiration.

Pour ce faire, les mèches de cheveux teints ont été immergées dans un cristallisoir recouvert d'un verre de montre et renfermant une solution de sueur synthétique de composition suivante :

- NaCl 1,0 g
- Hydrogénophosphate de potassium 0,1 g
- Histidine 0,025 g
- Acide lactique q.s. pH 3,2
- Eau distillée q.s.p. 100 g

On a laissé séjourner les mèches de cheveux teints dans cette solution de sueur synthétique pendant 48 heures à 37° C. Les mèches ont ensuite été rincées puis séchées.

La couleur des mèches a été ensuite évaluée à nouveau dans le système MUNSELL au moyen d'un colorimètre CM 2002 MINOLTA ®.

La différence entre la couleur de la mèche avant le test de résistance à la transpiration et la couleur de la mèche après le test de résistance à la transpiration a été calculée en appliquant la formule de NICKERSON :

$$\Delta E = 0{,}4C_0 dH + 6dV + 3dC$$

telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et $C_0$ représente la pureté de la mèche par rapport à laquelle on désire évaluer la différence de couleur.

La dégradation de la couleur est d'autant plus forte que la valeur de ΔE est élevée.

Les résultats sont donnés dans le tableau ci-dessous :

| **EXEMPLE** | Couleur des cheveux avant le test | Couleur des cheveux après le test | **Dégradation de la couleur** | | | |
|---|---|---|---|---|---|---|
| | | | ΔH | ΔV | ΔC | **ΔE** |
| 1 (*) | 9.2 RP 3.3 / 2.6 | 2.5 YR 3.9 / 2.1 | 13.3 | 0.6 | 0.5 | 18.9 |
| 2 | 2.6 RP 3.0 / 3.7 | 6.1 RP 3.5 / 3.2 | 3.5 | 0.5 | 0.5 | 9.7 |
| 3 | 2.6 RP 3.0 / 2.9 | 4.1 RP 3.0 / 2.9 | 1.5 | 0 | 0 | 1.7 |
| 4 | 2.7 RP 3.2 / 3.2 | 6.1 RP 3.5 / 3.0 | 3.4 | 0.3 | 0.2 | 6.8 |

Ces résultats montrent que la coloration obtenue en mettant en oeuvre la composition tinctoriale de l'exemple 1 ne faisant pas partie de l'invention car contenant l'association d'un diamino pyrazole et d'un méta-aminophénol non halogéné, conduit à une coloration nettement moins résistante à l'action de la transpiration que les colorations obtenues en mettant en oeuvre les compositions des exemples 2 à 4, faisant toutes partie de l'invention car contenant l'association d'un diamino pyrazole et d'un méta-aminophénol halogéné en ortho du phénol.

**EXEMPLES DE TEINTURE 5 à 8**

On a préparé les compositions tinctoriales, conformes à l'invention, suivantes (teneurs en grammes) :

| **EXEMPLE** | **5** | **6** | **7** | **8** |
|---|---|---|---|---|
| 4,5-diamino pyrazole, 2 HCl (base d'oxydation) | 0,513 | - | - | - |
| 1-méthyl 4,5-diamino pyrazole, 2 HCl (base d'oxydation) | - | 0,555 | 0,555 | 0,555 |
| 3-amino 2-chloro 6-méthyl phénol (coupleur conforme à l'invention) | 0,473 | 0,473 | - | - |
| 3-amino 6-chloro phénol (coupleur conforme à l'invention) | - | - | 0,431 | - |
| 3-(β-aminoéthyl)amino 6-chloro phénol (coupleur conforme à l'invention) | - | - | - | 0,560 |
| Support de teinture commun | (**) | (**) | (**) | (**) |
| Eau déminéralisée q.s.p. | 100 g | 100 g | 100 g | 100 g |
| (**) support de teinture commun :<br>Il est identique à celui utilisé pour les exemples 1 à 4 ci-dessus. | | | | |

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances figurant dans le tableau ci-dessous :

| **EXEMPLE** | **NUANCE OBTENUE** |
|---|---|
| 5 | Cuivré rouge |
| 6 | Cuivré rouge |

(suite)

| EXEMPLE | NUANCE OBTENUE |
|---|---|
| 7 | Irisé rouge |
| 8 | Rouge irisé |

## Revendications

**1.** Composition pour la teinture d'oxydation des fibres kératiniques humaines et en particulier des fibres kératiniques humaines telles que les cheveux, **caractérisée par le fait qu'**elle comprend, dans un milieu approprié pour la teinture :

- au moins une base d'oxydation choisie parmi les diamino pyrazoles ;
- et au moins un coupleur choisi parmi les méta- aminophénols halogénés de formule (I) suivante, et leurs sels d'addition avec un acide :

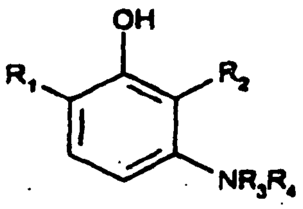

(I)

dans laquelle :

- $R_1$ et $R_2$, qui peuvent être identiques ou différents, représentent un atome d'hydrogène, un atome d'halogène tel que le chlore, le brome, l'iode ou le fluor, un radical alkyle en $C_1$-$C_4$, monohydroxyalkyle en $C_1$-$C_4$, polyhydroxyalkyle en $C_2$-$C_4$, alcoxy en $C_1$-$C_4$, monohydroxyalcoxy en $C_1$-$C_4$ ou polyhydroxyalcoxy en $C_2$-$C_4$ ;
- $R_3$ et $R_4$, qui peuvent être identiques ou différents, représentent un atome d'hydrogène, un radical alkyle en $C_1$-$C_4$, monohydroxyalkyle en $C_1$-$C_4$, polyhydroxyalkyle en $C_2$-$C_4$ ou monoaminoalkyle en $C_1$-$C_4$ ;

étant entendu qu'au moins un des radicaux $R_1$ et $R_2$ représente un atome d'halogène.

**2.** Composition selon la revendication 1, **caractérisée par le fait que** les méta-aminophénols halogénés de formule (I) sont choisis parmi le 3-amino 6-chloro phénol, le 3-amino 6-bromo phénol, le 3-(β-aminoéthyl)amino 6-chloro phénol, le 3-(β-hydroxyéthyl)amino 6-chloro phénol, le 3-amino 2-chloro 6-méthyl phénol, et leurs sels d'addition avec un acide.

**3.** Composition selon la revendication 1 ou 2, **caractérisée par le fait que** les diamino pyrazoles utilisables à titre de base d'oxydation sont choisis parmi :

a) les diamino pyrazoles de formule (II) suivante et leurs sels d'addition avec un acide :

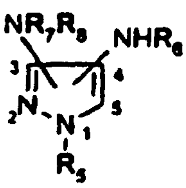

(II)

dans laquelle :

- $R_5$ représente un atome d'hydrogène, un radical alkyle en $C_1$-$C_6$, hydroxyalkyle en $C_2$-$C_4$, benzyle, phényle, benzyle substitué par un atome d'halogène ou par un groupe alkyle en $C_1$-$C_4$, ou alcoxy en $C_1$-$C_4$, ou forme avec l'atome d'azote du groupement $NR_7R_8$ en position 5 un hétérocycle héxahydropyridazinique ou tétrahydropyrazolique éventuellement monosubstitué par un groupe alkyle en $C_1$-$C_4$ ;
- $R_6$ et $R_7$, identiques ou différents, représentent un atome d'hydrogène, un radical alkyle en $C_1$-$C_4$, hydroxyalkyle en $C_2$-$C_4$, benzyle ou phényle ;
- $R_8$ représente un atome d'hydrogène, un radical alkyle en $C_1$-$C_6$ ou hydroxyalkyle en $C_2$-$C_4$ ; sous réserve que $R_6$ représente un atome d'hydrogène lorsque $R_5$ représente un radical benzyle substitué ou forme un hétérocycle avec l'atome d'azote du groupement $NR_7R_8$ en position 5 ;

b) les diamino pyrazoles de formule (III) suivante, et leurs sels d'addition avec un acide :

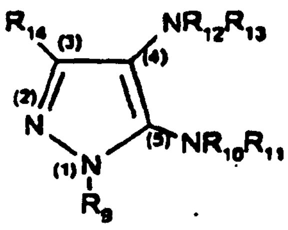

(III)

dans laquelle :

- $R_9$, $R_{10}$, $R_{11}$, $R_{12}$ et $R_{13}$, identiques ou différents, représentent un atome d'hydrogène ; un radical alkyle en $C_1$-$C_6$ linéaire ou ramifié ; un radical hydroxyalkyle en $C_2$-$C_4$ ; un radical aminoalkyle en $C_2$-$C_4$ ; un radical phényle ; un radical phényle substitué par un atome d'halogène ou un radical alkyle en $C_1$-$C_4$, alcoxy en $C_1$-$C_4$, nitro, trifluorométhyle, amino ou alkylamino en $C_1$-$C_4$ ; un radical benzyle ; un radical benzyle substitué par un atome d'halogène ou par un radical alkyle en $C_1$-$C_4$, alcoxy en $C_1$-$C_4$, méthylènedioxy ou amino ; ou un radical

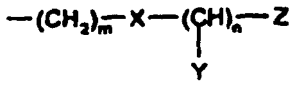

dans lequel m et n sont des nombres entiers, identiques ou différents, compris entre 1 et 3 inclusivement, X représente un atome d'oxygène ou bien le groupement NH, Y représente un atome d'hydrogène ou bien un radical méthyle, et Z représente un radical méthyle, un groupement OR ou NRR' dans lesquels R et R', qui peuvent être identiques ou différent, désignent un atome d'hydrogène, un radical méthyle ou un radical éthyle, étant entendu que lorsque $R_{10}$ représente un atome d'hydrogène, alors $R_{11}$ peut également représenter un radical amino ou alkylamino en $C_1$-$C_4$,
- $R_{14}$ représente un radical alkyle en $C_1$-$C_6$, linéaire ou ramifié ; un radical hydroxyalkyle en $C_1$-$C_4$ ; un radical aminoalkyle en $C_1$-$C_4$ ; un radical alkyle ($C_1$-$C_4$)aminoalkyle en $C_1$-$C_4$ ; un radical dialkyle ($C_1$-$C_4$)-aminoalkyle en $C_1$-$C_4$ ; un radical hydroxyalkyle ($C_1$-$C_4$)-amino alkyle en $C_1$-$C_4$ ; un radical alcoxy ($C_1$-$C_4$) méthyle ; un radical phényle ; un radical phényle substitué par un atome d'halogène ou par un radical alkyle en $C_1$-$C_4$, alcoxy en $C_1$-$C_4$, nitro, trifluorométhyle, amino ou alkylamino en $C_1$-$C_4$ ; un radical benzyle ; un radical benzyle substitué par un atome d'halogène ou par un radical alkyle en $C_1$-$C_4$, alcoxy en $C_1$-$C_4$, nitro, trifluorométhyle, amino ou alkylamino en $C_1$-$C_4$ ; un hétérocycle choisi parmi le thiophène, le furane et la pyridine, ou encore un radical $-(CH_2)_p-O-(CH_2)_q-OR''$, dans lequel p et q sont des nombres entiers, identiques ou différents, compris entre 1 et 3 inclusivement et R'' représente un atome d'hydrogène ou un radical méthyle,

étant entendu que dans la formule (III) ci-dessus :
- au moins un des radicaux $R_{10}$, $R_{11}$, $R_{12}$ et $R_{13}$ représente un atome d'hydrogène,
- lorsque que $R_{10}$, respectivement $R_{12}$, représente un radical phényle substitué ou non, ou un radical benzyle ou un radical

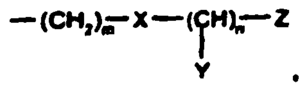

alors $R_{11}$, respectivement $R_{13}$, ne peut représenter aucun de ces trois radicaux,
- lorsque $R_{12}$ et $R_{13}$ représentent simultanément un atome d'hydrogène, alors $R_9$ peut former, avec $R_{10}$ et $R_{11}$, un hétérocycle hexahydropyrimidinique ou tétrahydroimidazolique éventuellement substitué par un radical alkyle en $C_1$-$C_4$ ou 1,2,4-tétrazolique,
- lorsque $R_{10}$, $R_{11}$, $R_{12}$ et $R_{13}$ représentent un atome d'hydrogène ou un radical alkyle en $C_1$-$C_6$, alors $R_9$ ou $R_{14}$ peut également représenter un reste hétérocyclique 2, 3 ou 4-pyridyle, 2 ou 3-thiényle, 2 ou 3-furyle éventuellement substitué par un radical méthyle ou bien encore un radical cyclohexyle.

**4.** Composition selon la revendication 3, **caractérisée par le fait que** les diamino pyrazoles de formule (II) sont choisis parmi le 4,5-diamino 1-(4'-méthoxybenzyle) pyrazole, le 4,5-diamino 1-(4'-méthylbenzyle) pyrazole, le 4,5-diamino 1-(4'-chlorobenzyle) pyrazole, le 4,5-diamino 1-(3'-méthoxybenzyle) pyrazole, le 4-amino 1-(4'-méthoxybenzyle) 5-méthylamino pyrazole, le 4-amino 5-(β-hydroxyéthyl)amino 1-(4'-méthoxybenzyle) pyrazole, le 4-amino 5-(β-hydroxyéthyt)amino 1-méthyl pyrazole, le 4-amino (3) 5-méthylamino pyrazole, le 3-(5),4-diamino pyrazole, le 4,5-diamino 1-méthyl pyrazole, le 4,5-diamino 1-benzyl pyrazole, la 3-amino 4,5,7,8-tétrahydro pyrazolo [1,5-a] pyrimidine, le 7-amino 2,3-dihydro 1-H-imidazol [1,2-b] pyrazole, la 3-amino 8-méthyl 4,5,7,8-tétrahydropyrazolo [1,5-a] pyrimidine, et leurs sels d'addition avec un acide.

**5.** Composition selon la revendication 3, **caractérisée par le fait que** les diamino pyrazoles de formule (III) sont choisis parmi :

- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino 1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino 3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphényl) pyrazole,
- le 4,5-diamino 3-méthyl 1-(3'-méthoxyphényl) pyrazole,
- le 4,5-diamino 3-méthyl 1-(4'-méthoxyphényl) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminométhyl 1-méthyl pyrazole,
- le 4,5-diamino 3-diméthylaminométhyl 1-éthyl pyrazole,
- le 4,5-diamino 3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,

- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phényl 3-méthyl pyrazole,
- le 4-amino 5-(β-hydroxyéthyl)amino 1-(2-méthoxyphényl) 3-méthyl pyrazole,
- le 4-amino 5-(β-hydroxyéthyl)amino 1-(3-méthoxyphényl) 3-mèthyl pyrazole,
- le 4-amino 5-(β-hydroxyéthyl)amino 1-(4-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 4,5-diamino 1,3-diméthyl pyrazole,
- le 4,5-diamino 3-tert.butyl 1-méthyl pyrazole,
- le 4,5-diamino 1-tert.butyl 3-méthyl pyrazole,
- le 4,5-diamino 1-méthyl 3-phényl pyrazole,
- le 4,5-diamino 1-(β-hydroxyéthyl) 3-méthyl pyrazole,
- le 4,5-diamino 1-(β-hydroxyéthyl) 3-phényl pyrazole,
- le 4,5-diamino 1-méthyl 3-(2'-chlorophényl) pyrazole,
- le 4,5-diamino 1-méthyl 3-(4'-chlorophényl) pyrazole,
- le 4,5-diamino 1-méthyl 3-(3'-trifluorométhylphényl) pyrazole,
- le 4,5-diamino 1,3-diphényl pyrazole,
- le 4,5-diamino 3-méthyl 1-phényl pyrazole,
- le 4-amino 1,3-diméthyl 5-phénylamino pyrazole,
- le 4-amino 1-éthyl 3-méthyl 5-phénylamino pyrazole,
- le 4-amino 1,3-diméthyl 5-méthylamino pyrazole,
- le 4-amino 3-méthyl 1-isopropyl 5-méthylamino pyrazole,
- le 4-amino 3-isobutoxyméthyl 1-méthyl 5-méthylamino pyrazole,
- le 4-amino 3-méthoxyéthoxyméthyl 1-méthyl 5-méthylamino pyrazole,
- le 4-amino 3-hydroxyméthyl 1-méthyl 5-méthylamino pyrazole,
- le 4-amino 1,3-diphényl 5-phénylamino pyrazole,
- le 4-amino 3-méthyl 5-méthylamino 1-phényl pyrazole,
- le 4-amino 1,3-diméthyl 5-hydrazino pyrazole,
- le 5-amino 3-méthyl 4-méthylamino 1-phényl pyrazole,
- le 5-amino 1-méthyl 4-(N,N-méthylphényl)amino 3-(4'-chlorophényl) pyrazole,
- le 5-amino 3-éthyl 1-méthyl 4-(N,N-méthylphényl)amino pyrazole,
- le 5-amino 1-méthyl 4-(N,N-méthylphényl)amino 3-phényl pyrazole,
- le 5-amino 3-éthyl 4-(N,N-méthylphényl)amino pyrazole,
- le 5-amino 4-(N,N-méthylphényl)amino 3-phényl pyrazole,
- le 5-amino 4-(N,N-méthylphényl)amino 3-(4'-méthylphényl) pyrazole,
- le 5-amino 3-(4'-chlorophényl) 4-(N,N-méthylphényl)amino pyrazole,
- le 5-amino 3-(4'-méthoxyphényl) 4-(N,N-méthylphényl)amino pyrazole,
- le 4-amino 5-méthylamino 3-phényl pyrazole,
- le 4-amino 5-éthylamino 3-phényl pyrazole,
- le 4-amino 5-éthylamino 3-(4'-méthylphényl) pyrazole,
- le 4-amino 3-phényl 5-propylamino pyrazole,
- le 4-amino 5-butylamino 3-phényl pyrazole,
- le 4-amino 3-phényl 5-phénylamino pyrazole,
- le 4-amino 5-benzylamino 3-phényl pyrazole,
- le 4-amino 5-(4'-chlorophényl)amino 3-phényl pyrazole,
- le 4-amino 3-(4'-chlorophényl) 5-phénylamino pyrazole,

- le 4-amino 3-(4'-méthoxyphényl) 5-phénylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoéthyl)amino 1,3-diméthyl pyrazole,

et leurs sels d'addition avec un acide.

**6.** Composition selon la revendication 5, **caractérisée par le fait que** les diamino pyrazoles de formule (III) sont choisis parmi :

- le 4,5-diamino 1,3-diméthyl pyrazole,
- le 4,5-diamino 3-méthyl 1-phényl pyrazole,
- le 4,5-diamino 1-méthyl 3-phényl pyrazole,
- le 4-amino 1,3-diméthyl 5-hydrazino pyrazole,
- le 1-benzyl 4,5-diamino 3-méthyl pyrazole,
- le 4,5-diamino 3-tert-butyl 1-méthyl pyrazole,
- le 4,5-diamino 1-tert-butyl 3-méthyl pyrazole,
- le 4,5-diamino 1-(β-hydroxyéthyl) 3-méthyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 1-éthyl 3-hydroxyméthyl pyrazole,
- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,

et leurs sels d'addition avec un acide.

**7.** Composition selon l'une quelconque des revendications précédentes, **caractérisée par le fait que** le ou les diamino pyrazoles et/ou le ou les sels d'addition avec un acide correspondants représentent de 0,0005 à 12 % en poids du poids total de la composition tinctoriale.

**8.** Composition selon la revendication 7, **caractérisée par le fait que** le ou les diamino pyrazoles et/ou le ou les sels d'addition avec un acide correspondants représentent de 0,005 à 6 % en poids du poids total de la composition tinctoriale.

**9.** Composition selon l'une quelconque des revendications précédentes, **caractérisée par le fait que** le ou les méta-aminophénols halogénés de formule (I) et/ou le ou les sels d'addition avec un acide correspondants représentent de 0,0001 à 5% en poids du poids total de la composition tinctoriale.

**10.** Composition selon la revendication 9, **caractérisée par le fait que** le ou les méta-aminophénols halogénés de formule (I) et/ou le ou les sels d'addition avec un acide correspondants représentent de 0,005 à 3 % en poids du poids total de la composition tinctoriale.

**11.** Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

**12.** Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**13.** Composition selon l'une quelconque des revendications précédentes, **caractérisée par le fait qu'**elle présente un pH compris entre 3 et12.

**14.** Composition selon l'une quelconque des revendications précédentes, **caractérisée par le fait qu'**elle se présente

sous forme de liquides, de crèmes, de gels, ou sous toute autre forme appropriée pour réaliser une teinture des fibres kératiniques, et notamment des cheveux humains.

**15.** Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 14, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

**16.** Procédé selon la revendication 15, **caractérisé par le fait que** l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides.

**17.** Dispositif à plusieurs compartiments, ou "kit" de teinture à plusieurs compartiments, dont un premier compartiment renferme une composition tinctoriale telle que définie à l'une quelconque des revendications 1 à 14 et un second compartiment renferme une composition oxydante.

## Claims

**1.** Composition for the oxidation dyeing of human keratin fibres and in particular human keratin fibres such as the hair, **characterized in that** it comprises, in a medium which is suitable for dyeing:

- at least one oxidation base chosen from diaminopyrazoles;
- and at least one coupler chosen from the halogenated meta- aminophenols of formula (I) below, and the addition salts thereof with an acid:

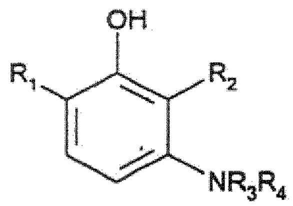

(I)

in which:

- $R_1$ and $R_2$, which may be identical or different, represent a hydrogen atom, a halogen atom such as chlorine, bromine, iodine or fluorine, a $C_1$-$C_4$ alkyl radical, a $C_1$-$C_4$ monohydroxyalkyl radical, a $C_2$-$C_4$ polyhydroxyalkyl radical, a $C_1$-$C_4$ alkoxy radical, a $C_1$-$C_4$ monohydroxyalkoxy radical or a $C_2$-$C_4$ polyhydroxyalkoxy radical;
- $R_3$ and $R_4$, which may be identical or different, represent a hydrogen atom, a $C_1$-$C_4$ alkyl radical, a $C_1$-C4 monohydroxyalkyl radical, a $C_2$-$C_4$ polyhydroxyalkyl radical or a $C_1$-$C_4$ monoaminoalkyl radical; it being understood that at least one of the radicals $R_1$ and $R_2$ represents a halogen atom.

**2.** Composition according to Claim 1, **characterized in that** the halogenated meta-aminophenols of formula (I) are chosen from 3-amino-6-chlorophenol, 3-Amino-6-bromophenol, 3-(β-aminoethyl) amino-6-chlorophenol, 3-(β-hydroxyethyl) mino-6-chlorophenol and 3-amino-2-chloro-6-methylphenol, and the addition salts thereof with an acid.

**3.** Composition according to Claim 1 or 2, **characterized in that** the diaminopyrazoles which can be used as oxidation bases are chosen from:

a) the diaminopyrazoles of formula (II) below, and the addition salts thereof with an acid:

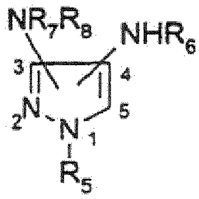

(II)

in which;

- $R_5$ represents a hydrogen atom, a $C_1$-$C_6$ alkyl radical, a $C_2$-$C_4$ hydroxyalkyl radical, a benzyl radical, a phenyl radical, a benzyl radical substituted with a halogen atom or with a $C_1$-$C_4$ alkyl Or $C_1$-C4 alkoxy group, or forms, with the nitrogen atom of the group $NR_7R_8$ in position 5, a hexahydropyridazine or tetrahydropyrazole heterocycle which is optionally monosubstituted with a $C_1$-$C_4$ alkyl group;
- $R_6$ and $R_7$, which may be identical or different, represent a hydrogen atom, a $C_1$-$C_4$ alkyl radical, a $C_2$-$C_4$ hydroxyalkyl radical, a benzyl radical or a phenyl radical; g
- $R_8$ represents a hydrogen atom, or a $C_1$-$C_6$ alkyl or $C_2$-$C_4$ hydroxyalkyl radical; with the proviso that $R_6$ represents a hydrogen atom when $R_5$ represents a substituted benzyl radical or forms a heterocycle with the nitrogen atom of the group $NR_7R_8$ in position 5;

b) the diaminopyrazoles of formula (III) below, and the addition salts thereof with an acid:

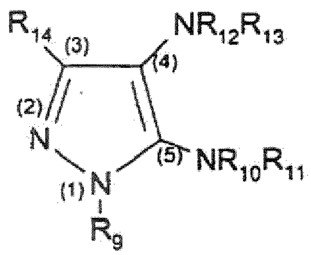

(III)

in which:

- $R_9$, $R_{10}$, $R_{11}$, $R_{12}$ and $R_{13}$, which may be identical or different, represent a hydrogen atom; a linear or branched $C_1$-$C_6$ alkyl radical; a $C_2$-$C_4$ hydroxyalkyl radical; a $C_2$-$C_4$ aminoalkyl radical; a phenyl radical; a phenyl radical substituted with a halogen atom or a $C_1$-$C_4$ alkyl, $C_1$-$C_4$ alkoxy, nitro, trifluoromethyl, amino or $C_1$-$C_4$ alkylamino radical; a benzyl radical; a benzyl radical substituted with a halogen atom or with a $C_1$-$C_4$ alkyl, $C_1$-C4 alkoxy, methylenedioxy or amino radical; or a radical

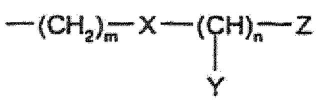

in which m and n are integers, which may be identical or different, between 1 and 3 inclusive, X represents an oxygen atom or an NH group, Y represents a hydrogen atom or a methyl radical, and Z represents a methyl radical, a group OR or NRR' in which R and R', which may be identical or different, denote a hydrogen atom, a methyl radical or an ethyl radical, it being understood that when $R_{10}$ represents a hydrogen atom, then $R_{11}$ can also represent an amino or $C_1$-$C_4$ alkylamino radical,
- $R_{14}$ represents a linear or branched $C_1$-$C_6$ alkyl radical; a $C_1$-$C_4$ hydroxyalkyl radical; a $C_1$-$C_4$ aminoalkyl radical; a $(C_1$-$C_4)$ alkylamino$(C_1$-$C_4)$alkyl radical; a di$(C_1$-$C_4)$ alkylamino$(C_1$-$C_4)$ alkyl radical; a hydroxy $(C_1$-$C_4)$alkylamino$(C_1$-$C_4)$ alkyl radical; a $(C_1$-$C_4)$alkoxymethyl radical; a phenyl radical; a phenyl radical

substituted with a halogen atom or with a $C_1$-$C_4$ alkyl, $C_1$-$C_4$ alkoxy, nitro, trifluoromethyl, amino or $C_1$-$C_4$ alkylamino radical; a benzyl radical; a benzyl radical substituted with a halogen atom or with a $C_1$-$C_4$ alkyl, $C_1$-$C_4$ alkoxy, nitro, trifluoromethyl, amino or $C_1$-$C_4$ alkylamino radical, a heterocycle chosen from thiophene, furan and pyridine, or alternatively a radical $-(CH_2)_p-O-(CH_2)_q-OR''$, in which p and q are integers, which may be identical or different, between 1 and 3 inclusive, and R" represents a hydrogen atom or a methyl radical, it being understood that, in formula (III) above,

- at least one of the radicals $R_{10}$, $R_{11}$, $R_{12}$ and $R_{13}$ represents a hydrogen atom,
- when $R_{10}$, or $R_{12}$, respectively, represents a substituted or unsubstituted phenyl radical, or a benzyl radical or a radical

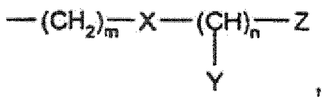

then $R_{11}$, or $R_{13}$, respectively, cannot represent any of these three radicals,
- when $R_{12}$ and $R_{13}$ simultaneously represent a hydrogen atom, then $R_9$ can form, with $R_{10}$ and $R_{11}$, a hexahydropyrimidine or tetrahydroimidazole heterocycle which is optionally substituted with a $C_1$-$C_4$ alkyl or 1,2,4-tetrazole radical,
- when $R_{10}$, $R_{11}$, $R_{12}$ and $R_{13}$ represent a hydrogen atom or a $C_1$-$C_6$ alkyl radical, then $R_9$ or $R_{14}$ can also represent a 2-, 3- or 4-pyridyl, 2- or 3-thienyl or 2- or 3-furyl heterocyclic residue which is optionally substitute with a methyl radical or alternatively a cyclohexyl radical.

**4.** Composition according to Claim 3, **characterized in that** the diaminopyrazoles of formula (II) are chosen from 4,5-diamino-1-(4'-methoxybenzyl)pyrazole, 4,5-diamino-1-(4'-methylbenzyl)pyrazole, 4,5-diamino-1-(4'-chlorobenzyl) pyrazole, 4,5-diamino-1-(3'-methoxybenzyl)pyrazole, 4-amino-1-(4'-methoxybenzyl)-5-methylaminopyrazole, 4-amino-5-(β-hydroxyethyl)amino-1-(4'-methoxybenzyl)pyrazole, 4-amino-5-(β-hydroxyethyl)-amino-1-methylpyrazole, 4-Amino-(3)5-methylaminopyrazole, 3-(5)4-diaminopyrazole, 4,5-diamino-1-methylpyrazole, 4,5-diamino-1-benzylpyrazole, 3-amino-4,5,7,8-tetrahydropyrazolo[1,5-a]pyrimidine, 7-amino-2,3-dihydro-1H-imidazol[1,2-b] pyrazole and 3-amino-8-methyl-4,5,7,8-tetrahydropyrazolo[1,5-a]pyrimidine, and the addition salts thereof with an acid.

**5.** Composition according to Claim 3, **characterized in that** the diaminopyrazoles of formula (III) are chosen from:

- 1-benzyl-4,5-diamino-3-methylpyrazole,
- 4,5-diamino-1-(β-hydroxyethyl-3-(4'-methoxyphenyl)-pyrazole,
- 4,5-diamino-1-(-hydroxyethyl)-3-(4'-methylphenyl)-pyrazole,
- 4,5-diamino-1-(β-hydroxyethyl)-3-(3'-methylphenyl)-pyrazole,
- 4,5-diamino-3-methyl-1-isopropylpyrazole,
- 9,5-diamino-3-(4'-methoxyphenyl-1-isopropylpyrazole,
- 4,5-diamino-1-ethyl-3-methylpyrazole,
- 4,5-diamino-1-ethyl-3-(4'-methoxyphenyl)pyrazole,
- 4,5-diamino-3-hydroxymethyl-1-methylpyrazole,
- 4,5-diamino-1-ethyl-3-hydroxymethylpyrazole,
- 4,5-diamino-3-hydroxymethyl-1-isopropylpyrazole,
- 4,5-diamino-3-hydroxymethyl-1-tert-butylpyrazole,
- 9,5-diamino-3-hydroxymethyl-1-phenylpyrazole,
- 4,5-diamino-3-hydroxymethyl-1-(2'-methoxyphenyl)-pyrazole,
- 4,5-diamino-3-hydroxymethyl-1-(3'-methoxyphenyl)-pyrazole,
- 4,5-diamino-3-hydroxymethyl-1-(4'-methoxyphenyl)-pyrazole,
- 1-benzyl-4,5-diamino-3-hydroxymethylpyrazole,
- 4,5-diamino-3-methyl-1-(2'-methoxyphenyl)pyrazole,
- 4,5-diamino-3-methyl-1-(3'-methoxyphenyl)pyrazole,
- 4,5-diamino-3-methyl-1-(4'-methoxyphenyl)pyrazole,
- 3-aminomethyl-9,5-diamino-1-methylpyrazole,
- 3-aminomethyl-4,5-diamino-1-ethylpyrazole,

- 3-aminomethyl-4,5-diamino-1-isopropylpyrazole,
- 3-aminomethyl-4,5-diamino-1-tert-butylpyrazole,
- 9,5-diamino-3-dimethylaminomethyl-1-ethylpyrazole,
- 9,5-diamino-3-dimethylaminomethyl-1-methylpyrazole,
- 9,5-diamino-3-dimethylaminomethyl-1-isopropylpyrazole,
- 4,5-diamino-3-dimethylaminomethyl-1-tert-butylpyrazole,
- 9,5-diamino-3-ethylaminomethyl-methylpyrazole,
- 9,5-diamino-3-ethylaminomethyl-1-ethylpyrazole,
- 4,5-diamino-3-ethylaminomethyl-1-isopropylpyrazole,
- 4,5-diamino-3-ethylaminomethyl-1-tert-butylpyrazole,
- 4,5-diamino-3-methylaminomethyl-1-methylpyrazole,
- 4,5-diamino-3-methylaminomethyl-1-isopropylpyrazole,
- 4,5-diamino-1-ethyl-3-methylamanomethylpyrazole,
- 1-tert-butyl-4,5-diamino-3-methylaminomethylpyrazole,
- 4,5-diamino-3-[(β-hydroxyethyl)aminomethyl]-1-methylpyrazole,
- 4,5-diamino-3-[(β-hydroxyethyl)aminomethy]-1-isopropylpyrazole,
- 4,5-diamino-1-ethyl-3-[(β-hydroxyethyl)aminomethyl]-pyrazole,
- 1-tert-butyl-4,5-diamino-3-[(β-hydroxyethy)aminomethyl]pyrazole,
- 4-amino-5-(β-hydroxyethyl)amino-1,3-dimethylpyrazole,
- 4-amino-5(β-hydroxyethyl)amino-1-isopropyl-3-methylpyrazole,
- 4-amino-5-(β-hydroxyethyl)amino-1-ethyl-3-methylpyrazole,
- 4-amino-5-(β-hydroxyethyl)amino-1-tert-butyl-3-methylpyrazole,
- 4-amino-5-(β-hydroxyethyl)amino-1-phenyl-3-methylpyrazole,
- 4-amino-5-(β-hydroxyethyl)amino-1-2-methoxyphenyl)-3-methylpyrazole,
- 4-amino-5-(β-hydroxyethyl)amino-1-(3-methoxyphenyl)-3-methylpyrazole,
- 4-amino-5-(β-hydroxyethyl)amino-1-4-methoxyphenyl)-3-methylpyrazole,
- 4-amino-5-(β-hydroxyethyl)amino-1-benzyl-3-methylpyrazole,
- 4-amino-1-ethyl-3-methyl-5-methylaminopyrazole,
- 4-amino-1-tert-butyl-3-methyl-5-methylaminopyrazole,
- 4,5-diamino-1,3-dimethylpyrazole,
- 4,5-diamino-3-tert-butyl-1-methylpyrazole,
- 4,5-diamino-1-tert-butyl-3-methylpyrazole,
- 4,5-diamino-1-methyl-3-phenylpyrazole,
- 4,5-diamino-1-(β-hydroxyethyl)-3-methylpyrazole,
- 4,5-diamino-1-(β-hydroxyethyl)-3-phenylpyrazole,
- 4,5-diamino-1-methyl-3-(2'-chlorophenyl)pyrazole,
- 4,5-diamino-1-methyl-3-(4'-chlorophenyl)pyrazole,
- 4,5-diamino-1-methyl-3-(3'-trifluoromethylphenyl)-pyrazole,
- 4,5-diamino-1,3-diphenylpyrazole,
- 4,5-diamino-3-methyl-1-phenylpyrazole,
- 4-amino-1,3-dimethyl-5-phenylaminopyrazole,
- 4-amino-1-ethyl-3-methyl-5-phenylaminopyrazole,
- 4-amino-1,3-dimethyl-5-methylaminopyrazole,
- 4-amino-3-methyl-1-isopropyl-5-methylaminopyrazole,
- 4-amino-3-isobutoxymethyl-1-methyl-5-methylaminopyrazole,
- 4-amino-3-methoxyethoxymethyl-1-methyl-5-methylaminopyrazole,
- 4-amino-3-hydroxymethyl-1-methyl-5-methylaminopyrazole,
- 4-amino-1,3-diphenyl-5-phenylaminopyrazole,
- 4-amino-3-methyl-5-methylamino-1-phenylpyrazole,
- 4-amino-1,3-dimethyl-5-hydrazinopyrazole,
- 5-amino-3-methyl-4-methylamino-1-phenylpyrazole,
- 5-amino-1-methyl-4-N,N-methylphenyl)amino-3-(4'-chlorophenyl)pyrazole,
- 5-amino-3-ethyl-1-methyl-4-(N,N-methylphenyl)aminopyrazole,
- 5-amino-1-methyl-4-(N,N-methylphenyl)amino-3-phenylpyrazole,
- 5-amino-3-ethyl-4-(N,N-methylphenyl)aminopyrazole,
- 5-amino-4-(N,N-methylphenyl)amino-3-phenylpyrazole,
- 5-amino-4-(N,N-methylphenyl)amino-3-(4'-methylphenyl)pyrazole,
- 5-amino-3-(4'-chlorophenyl-4-(N,N-methylpheny)-aminopyrazole,

- 5-amino-3-(4'-methoxyphenyl)-4-(N,N-methylphenyl)-aminopyrazole,
- 4-amino-5-methylamino-3-phenylpyrazole,
- 4-amino-5-ethylamino-3-phenylpyrazole,
- 4-amino-5-ethylamino-3-(4'-methylphenyl)pyrazole,
- 4-amino-3-phenyl-5-propylaminopyrazole,
- 4-amino-5-butylamino-3-phenylpyrazole,
- 4-amino-3-phenyl-5-phenylaminopyrazole,
- 4-amino-5-benzylamino-3-phenylpyrazole,
- 4-amino-5-(4'-chlorophenyl)amino-3-phenylpyrazole,
- 4-amino-3-(4'-chlorophenyl)-5-phenylaminopyrazole,
- 4-amino-3-(4'-methoxyphenyl)-5-phenylaminopyrazole,
- 1-(4'-chlorobenzyl)-4,5-diamino-3-methylpyrazole,
- 4,5-diamino-3-hydroxymethyl-1-isopropylpyrazole,
- 4-amino-1-ethyl-3-methyl-5-methylaminopyrazole,
- 4-amino-5-(2'-aminoethyl)amino-1,3-dimethylpyrazole, and the addition salts thereof with an acid.

**6.** Composition according to Claim 5, **characterized in that** the diaminopyrazoles of formula (III) are chosen from:

- 4,5-diamino-1,3-dimethylpyrazole,
- 4,5-diamino-3-methyl-1-phenylpyrazole,
- 4,5-diamino-1-methyl-3-phenylpyrazole,
- 4-amino-1,3-dimethyl-5-hydrazinopyrazole,
- 1-benzyl-4,5-diamino-3-methylpyrazole,
- 4,5-diamino-3-tert-butyl-1-methylpyrazole,
- 4,5-diamino-1-tert-butyl-3-methylpyrazole,
- 4,5-diamino-1-(β-hydroxyethyl-3-methylpyrazole,
- 4,5-diamino-1-ethyl-3-methylpyrazole,
- 4,5-diamino-1-ethyl-3-(4'-methoxyphenyl)pyrazole,
- 4,5-diamino-1-ethyl-3-hydroxymethylpyrazole,
- 4,5-diamino-3-hydroxymethyl-1-methylpyrazole,
- 4,5-diamino-3-hydroxymethyl-1-isopropylpyrazole,
- 4,5-diamino-3-methyl-1-isopropylpyrazole,
- 4-amino-5-(2'-aminoethyl)amino-1,3-dimethylpyrazole,

and the addition salts thereof with an acid.

**7.** Composition according to any one of the preceding claims, **characterized in that** the diaminopyrazole(s) and/or the corresponding addition salt(s) with an acid represent(s) from 0.0005 to 12% by weight relative to the total weight of the dye composition.

**8.** Composition according to Claim 7, **characterized in that** the diaminopyrazole(s) and/or the corresponding addition salt(s) with an acid represent(s) from 0.005 to 6% by weight relative to the total weight of the dye composition.

**9.** Composition according to any one of the preceding claims, **characterized in that** the halogenated meta-aminophenol (s) of formula (I) and/or the corresponding addition salt(s) with an acid represent(s) from 0.0001 to 5% by weight relative to the total weight of the dye composition.

**10.** Composition according to Claim 9, **characterized in that** the halogenated meta-aminophenol(s) of formula (I) and/or the corresponding addition salt(s) with an acid represent(s) from 0.005 to 3% by weight relative to the total weight of the dye composition.

**11.** Composition according to any one of the preceding claims, **characterized in that** the addition salts with an acid are chosen from the hydrochlorides, hydrobromides, sulphates, tartrates, lactates and acetates.

**12.** Composition according to any one of the preceding claims, **characterized in that** the medium which is suitable for dyeing (or support) consists of water or of a mixture of water and at least one organic solvent chosen from $C_1$-$C_4$ lower alkanols, glycerol, glycols and glycol ethers, aromatic alcohols, similar products and mixtures thereof.

13. Composition according to any one of the preceding claims, **characterized in that** it has a pH of between 3 and 12.

14. Composition according to any one of the preceding claims, **characterized in that** it is in the form of liquids, creams or gels or in any other form which is suitable for dyeing keratin fibres, and in particular human hair.

15. Process for dyeing keratin fibres, and in particular human keratin fibres such as the hair, **characterized in that** at least one dye composition as def ined in any one of Claims 1 to 14 is applied to these fibres, and **in that** the colour is developed at acidic, neutral or alkaline pH with the aid of an oxidizing agent which is added to the dye composition just at the time of use, or which is present in an oxidizing composition that is applied simultaneously or sequentially.

16. Process according to Claim 15, **characterized in that** the oxidizing agent present in the oxidizing composition is chosen from hydrogen peroxide, urea peroxide, alkali metal bromates, persalts such as perborates, percarbonates and persulphates, and peracids.

17. Multi-compartment device or multi-compartment dyeing "kit", a first compartment of which contains a dye composition as defined in any one of Claims 1 to 14, and a second compartment of which contains an oxidizing composition.

**Patentansprüche**

1. zuaammensetzung zum oxidativen Färben von menschlichen Keratinfasern und insbesondere menschlichen Keratinfasern, wie Haaren, **dadurch gekennzeichnet, dass** sie in einem zum Färben geeigneten Träger enthält:

- mindestens eine Oxidationsbase, die unter den Diaminopyrazolen ausgewählt ist, und
- mindestens einen Kuppler, der unter den halogenierten m-Aminophenolen der folgenden Formel (I) und deren Additionssalzen mit einer Säure ausgewählt ist.

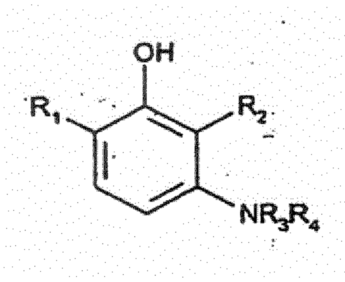

(I)

worin bedeuten:

- die Gruppen $R_1$ und $R_2$, die gleich oder verschieden sein können, ein Wasserstoffatom, ein Halogenatom, das unter Chlor, Brom, Iod oder Fluor ausgewählt ist, $C_{1-4}$-Alkyl, $C_{1-4}$-Monohydroxyalkyl, $C_{2-4}$-Polyhydroxyalkyl, $C_{1-4}$-Alkoxy, $C_{1-4}$-Monohydroxyalkoxy oder $C_{2-4}$-Polyhydroxyalkoxy,
- die Gruppen $R_3$ und $R_4$, die gleich oder verschieden sein können, ein Wasserstoffatom, $C_{1-4}$Alkyl, $C_{1-4}$-Monohydroxyalkyl, $C_{2-4}$-Polyhydroxyalkyl oder $C_{1-4}$-Monoaminoalkyl,
- mit der Maßgabe, dass mindestens eine der Gruppen $R_1$ und $R_2$ ein Halogenatom bedeutet.

2. zusammensetzung nach Anspruch 1, **dadurch gekennzeichnet, dass** die halogenierten m-aminophenole der Formel (I) unter 3-Amino-6-chlor-phenol, 3-amine-6-brom-phenol, 3-(β-Aminoethyl)amino-6-chlor-phenol, 3-(β-Hydroxyethyl)amino-6-chlorphenol, 3-Amino-2-chlor-6-methyl-phenol und deren Additionssalzen mit einer Säure ausgewählt sind.

3. Zusammensetzung nach Anspruch 1 oder 2, **dadurch gekennzeichnet, dass** die als Oxidationsbasen verwendbaren Diaminopyrazole ausgewählt sind unter:

a) Diaminopyrazolen der folgenden Formel (II) und deren Additionssalze mit einer Säure:

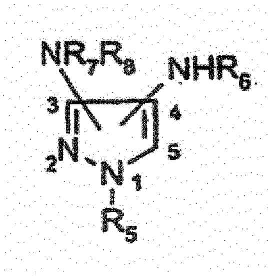

(II)

worin bedeuten:

- die Gruppe $R_5$ ein Wasserstoffatom, $C_{1-6}$-Alkyl, $C_{2-4}$-Hydroxyalkyl, Benzyl, Phenyl, eine mit einem Halogenatom oder einer $C_{1-4}$-Alkylgruppe substituierte Benzylgruppe oder $C_{1-4}$-Alkoxy oder sie bildet mit dem Stickstoffatom der Gruppe $NR_7R_8$ in 5-Stellung einen Hexahydropyridazin-Heterocyclus oder einem Tetrahydropyrazol-Heterocyclus, der gegebenenfalls mit einer $C_{1-4}$-Alkylgruppe monosubstituiert ist;
- die Gruppen $R_6$ und $R_7$, die gleich oder verschieden sind, ein Wasserstoffatom, $C_{1-4}$-Alkyl, $C_{2-4}$-Hydroxyalkyl, Benzyl oder Phenyl,
- die Gruppe $R_8$ ein Wasserstoffatom, $C_{1-6}$-Alkyl oder $C_2$-4-Hydroxyalkyl, mit der Maßgabe, dass $R_6$ Wasserstoff bedeutet, wenn $R_5$ einen substituierten Benzolring bedeutet oder mit dem Stickstoffatom der Gruppe $NR_7R_8$ in 5-Stellung einen Heterocyclus bildet;

b) die Diaminopyrazole der folgenden Formel (III) und deren Additionssalze mit einer Säure:

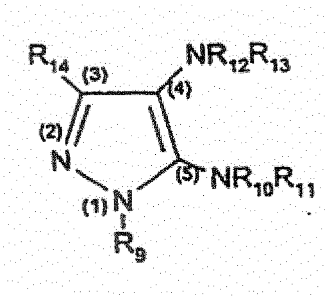

(III)

worin bedeuten:

- die Gruppen $R_9$, $R_{10}$, $R_{11}$, $R_{12}$ und $R_{13}$, die gleich oder verschieden sind, ein Wasserstoffatom; eine geradkettige oder verzweigte $C_{1-6}$-Alkylgruppe; $C_{2-4}$-Hydroxyalkyl; $C_{2-4}$-Aminoalkyl; Phenyl; eine Phenylgruppe, die mit einem Halogenatom oder $C_{1-4}$-Alkyl, $C_{1-4}$-Alkoxy, Nitro, Trifluormethyl, Amino oder $C_{1-4}$-Alkylamino substituiert ist; Benzyl; eine Benzylgruppe, die mit einem Halogenatom oder $C_{1-4}$-Alkyl, $C_{1-4}$-Alkoxy, Methylendioxy oder Amino substituiert ist; eine Gruppe

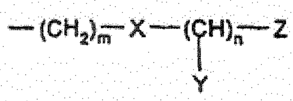

worin m und n, die gleich oder verschieden sind, ganze Zahlen von 1 bis 3 bedeuten, X ein Sauerstoffatom oder die Gruppe NH bedeutet, Y ein Wasserstoffatom oder die Methylgruppe ist und Z eine Methylgruppe, OR oder NRR' bedeutet, wobei R und R', die gleich oder verschieden sein können, ein Wasserstoffatom oder Methyl oder Ethyl bedeuten; mit der Maßgabe, dass, wenn $R_{10}$ Wasserstoff bedeutet, $R_{11}$ auch Amino oder $C_{1-4}$-Alkylamino bedeuten kann;
- $R_{14}$ eine geradkettige oder verzweigte $C_{1-6}$-Alkylgruppe; $C_{1-4}$-Hydroxyalkyl; $C_{1-4}$-Aminoalkyl; Alkyl($C_{1-4}$) aminoalkyl($C_{1-4}$) ; Dialkyl($C_{1-4}$)aminoalkyl($C_{1-4}$) ; Hydroxyalkyl($C_{1-4}$)aminoalkyl($C_{1-4}$); Alkoxy($C_{1-4}$)methyl; Phenyl; eine Phenylgruppe, die mit einem Halogenatom oder Alkyl($C_{1-4}$), Alkoxy($C_{1-4}$), Nitro, Trifluormethyl, Amino oder Alkyl($C_{1-4}$)amino substituiert ist; Benzyl; eine Benzylgruppe, die mit einem Halogenatom oder

Alkyl($C_{1-4}$), Alkoxy($C_{1-4}$), Nitro, Trifluormethyl, Amino oder Alkyl($C_{1-4}$)amino substituiert ist; einen Heterocyclus, der unter Thiophen, Furan und Pyridin ausgewählt ist, oder eine Gruppe $(CH_2)_p$-O-$(CH_2)_q$-OR", wobei p und q, die gleich oder voneinander verschieden sind, ganze Zahlen im Bereich von 1 bis 3 bedeuten und R" Wasserstoff oder Methyl bedeutet,

mit der Maßgabe, dass in der Formel (III) :

- mindestens eine der Gruppen $R_{10}$, $R_{11}$, $R_{12}$ und $R_{13}$ ein Wasserstoffatom bedeutet,
- wenn $R_{10}$ bzw. $R_{12}$ eine substituierte oder unsubstituierte Phenylgruppe oder eine Benzylgruppe oder eine Gruppe

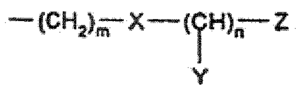

bedeutet, $R_{11}$ bzw. $R_{13}$ keine dieser drei Gruppen bedeuten kann,
- wenn $R_{12}$ und $R_{13}$ gleichzeitig Wasserstoff bedeuten, $R_9$ mit $R_{10}$ und $R_{11}$ einen Hexahydropyrimidin-Heterocyclus oder einen Tetrahydroimidazol-Heterocyclus bilden kann, der gegebenenfalls substituiert ist mit einer $C_{1-4}$-Alkylgruppe, oder 1,2,4-Tetrazol,
- wenn $R_{10}$, $R_{11}$, $R_{12}$ und $R_{13}$ ein Wasserstoffatom oder eine $C_{1-6}$-Alkylgruppe bedeuten, $R_9$ oder $R_{14}$ eine heterocyclische 2-, 3- oder 4-Pyridylgruppe, 2- oder 3-Thienylgruppe oder 2-oder 3-Furylgruppe bedeuten kann, die gegebenenfalls mit Methyl oder Cyclohexyl substituiert ist.

**4.** Zusammensetzung nach Anspruch 3, **dadurch gekennzeichnet, dass** die Diaminopyrazole der Formel (II) unter 4,5-Diamino-1-(4'-methoxybenzyl)-pyrazol, 4,5-Diamino-1-(4'-methylbenzyl)-pyrazol, 4,5-Diamino-1-(4'-chlorbenzyl)-pyrazol, 4,5-Diamino-1-(3'-methoxybenzyl)-pyrazol, 4-Amino-1-(4'-methoxybenzyl)-5-methylamino-parazol, 4-Amino-5-(β-hydroxyethyl)amino-1-(4'-methoxybenzyl)-pyrazol, 4-Amino-5-(β-hydroxyethyl)-amino-1-methyl-pyrazol, 4-Amino-(3)5-methylamino-pyrazol, 3(5),4-Diamino-pyrazol, 4,5-Diamino-1-methyl-pyrazol, 4,5-Diamino-1-benzyl-pyrazol, 3-Amino-4,5,7,8-tetrahydro-pyrazolo[1,5-a]-pyrimidin, 7-amino-2,3-dihydro-1H-imidazol[1,2-b]pyrazol, 3-Amino-8-methyl-4,5,7-8-tetrahydropyrazolo[1,5-a]pyrimidin und deren Additionssalzen mit einer Säure ausgewählt sind.

**5.** Zusammensetzung nach Anspruch 3, **dadurch gekennzeichnet, dass** die Diaminopyrazole der Formel (III) unter den folgenden Verbindungen ausgewählt sind:

- 1-Benzyl-4,5-diamino-3-methyl-pyrazol,
- 4,5-Diamino-1-(β-hydroxyethyl)-3-(4'-methoxyphenyl)-pyrazol,
- 4,5-Diamino-1-(β-hydroxyethyl)-3-(4'-methylphenyl)-pyrazol,
- 4,5-Diamino-1-(β-hydroxyethyl)-3-(3'-methylphenyl)-pyrazol,
- 4,5-Diamino-3-methyl-1-isopropyl-pyrazol,
- 4,5-Diamino-3-(4'-methoxyphenyl)-1-isopropyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-3-hydroxymethyl-1-methyl-pyrazol,
- 4,5-Diamino-1-ethyl-3-hydroxymethyl-pyrazol,
- 4,5-Diamino-3-hydroxymethyl-1-isopropyl-pyrazol,
- 4,5-Diamino-3-hydroxymethyl-1-t-butyl-pyrazol,
- 4,5-Diamino-3-hydroxymethyl-1-phenyl-pyrazol,
- 4,5-Diamino-3-hydroxymethyl-1-(2'-methoxyphenyl)-pyrazol,
- 4,5-Diamino-3-hydroxymethyl-1-(3'-methoxyphenyl)-pyrazol,
- 4,5-Diamino-3-hydroxymethyl-1-(4'-methoxy-phenyl)-pyrazol,
- 1-Benzyl-4,5-diamino-3-hydroxymethyl-pyrazol,
- 4,5-Diamino-3-methyl-1-(2'-methoxyphenyl)-pyrazol,
- 4,5-Diamino-3-methyl-1-(3'-methoxyphenyl)-pyrazol,
- 4,5-Diamino-3-methyl-1-(4'-methoxyphenyl)-pyrazol,

- 3-Aminomethyl-4,5-diamino-1-methyl-pyrazol,
- 3-Aminomethyl-4,5-diamino-1-ethyl-pyrazol,
- 3-Aminomethyl-4,5-diamino-1-isopropyl-pyrazol,
- 3-Aminomethyl-4,5-diamino-1-t-butyl-pyrazol,
- 4,5-Diamino-3-dimethylaminomethyl-1-methyl-pyrazol,
- 4,5-Diamino-3-dimethylaminomethyl-1-ethyl-pyrazol,
- 4,5-Diamino-3-dimethylaminomethyl-1-isopropyl-pyrazol,
- 4,5-Diamino-3-dimethylaminomethyl-1-t-butyl-pyrazol,
- 4,5-Diamino-3-ethylaminomethyl-1-methyl-pyrazol,
- 4,5-Diamino-3-ethylaminomethyl-1-ethyl-pyrazol,
- 4,5-Diamino-3-ethylaminomethyl-1-isopropyl-pyrazol,
- 4,5-Diamino-3-ethylaminomethyl-1-t-butyl-pyrazol,
- 4,5-Diamino-3-methylaminomethyl-1-methyl-pyrazol,
- 4,5-Diamino-3-methylaminomethyl-1-isopropyl-pyrazol,
- 4,5-Diamino-1-ethyl-3-methylaminomethyl-pyrazol,
- 1-t-Butyl-4,5-diamino-3-methylaminomethyl-pyrazol,
- 4,5-Diamino-3-[(β-hydroxyethyl)aminomethyl]-1-methyl-pyrazol,
- 4,5-Diamino-3-[(β-hydroxyethyl)aminomethyl]-1-isopropyl-pyrazol,
- 4,5-Diamino-1-ethyl-3-[(β-hydroxyethyl)aminomethyl]-pyrazol,
- 1-t-Butyl-4,5-diamino-3-[(β-hydroxyethyl)aminomethyl]-pyrazol,
- 4-Amino-5-(β-hydroxyethyl)amino-1,3-dimethyl-pyrazol,
- 4-Amino-5-(β-hydroxyethyl)amino-1-isopropyl-3-methyl-pyrazol,
- 4-Amino-5-(β-hydroxyethyl)amino-1-ethyl-3-methyl-pyrazol,
- 4-Amino-5-(β-hydroxyethyl)amino-1-t-butyl-3-methyl-pyrazol,
- 4-Amino-5-(β-hydroxyethyl)amino-1-phenyl-3-methyl-pyrazol,
- 4-Amino-5-(β-hydroxyethyl)amino-1-(2-methoxyphenyl)-3-methyl-pyrazol,
- 4-Amino-5-(β-hydroxyethyl)amino-l-(3-methoxyphenyl)-3-methyl-pyrazol,
- 4-Amino-5-(β-hydroxyethyl)amino-1-(4-methoxyphenyl)-3-methyl-pyrazol,
- 4-Amino-5-(β-hydroxyethyl)amino-1-benzyl-3-methyl-pyrazol,
- 4-Amino-1-ethyl-3-methyl-5-methylamino-pyrazol,
- 4-Amino-1-t-butyl-3-methyl-5-methylamino-pyrazol,
- 4,5-Diamino-1,3-dimethyl-pyrazol,
- 4,5.Diamino-3-t-butyl-1-methyl-pyrazol,
- 4,5-Diamino-1-t-butyl-3-methyl-pyrazol,
- 4,5-Diamino-1-methyl-3-phenyl-pyrazol,
- 4,5-Diamino-1-(β-hydroxyethyl)-3-methyl-pyrazol,
- 4,5-Diamino-1-(β-hydroxyethyl)-3-phenyl-pyrazol,
- 4,5-Diamino-1-methyl-3-(2'-chlorphenyl)-pyrazol,
- 4,5-Diamino-1-methyl-3-(4'-chlorphenyl)-pyrazol,
- 4,5-Diamino-1-methyl-3-(3'-trifluormethylphenyl)-pyrazol,
- 4,5-Diamino-1,3-diphenyl-pyrazol,
- 4,5-Diamino-3-methyl-1-phenyl-pyrazol,
- 4-Amino-1,3-dimethyl-5-phenylamino-pyrazol,
- 4-Amino-1-ethyl-3-methyl-5-phenylamino-pyrazol,
- 4-Amino-1,3-dimethyl-5-methylamino-pyrazol,
- 4-Amino-3-methyl-1-isopropyl-5-methylamino-pyrazol,
- 4-Amino-3-isobutoxymethyl-1-methyl-5-methylamino-pyrazol,
- 4-Amino-3-methoxyethoxymethyl-1-methyl-5-methylamino-pyrazol,
- 4-Amino-3-hydroxymethyl-1-methyl-5-methylamino-pyrazol,
- 4-Amino-1,3-diphenyl-5-phenylamino-pyrazol,
- 4-Amino-3-methyl-5-methylamino-1-phenyl-pyrazol,
- 4-Amino-1,3-dimethyl-5-hydrazino-pyrazol,
- 5-Amino-3-methyl-4-methylamino-1-phenyl-pyrazol,
- 5-Amino-1-methyl-4-(N,N-methylphenyl)-amino-3-(4'-chlorphenyl)-pyrazol,
- 5-Amino-3-ethyl-1-methyl-4-(N,N-methylphenyl)amino-pyrazol,
- 5-Amino-1-methyl-4-N,N-methylphenyl)amino-3-phenyl-pyrazol,
- 5-Amino-3-ethyl-4-(N,N-methylphenyl)amino-pyrazol,
- 5-Amino-4-(N,N-methylphenyl)amino-3-phenyl-pyrazol,

- 5-Amino-4-(N,N-methylphenyl)amino-3-(4'-methylphenyl)-pyrazol,
- 5-Amino-3-(4'-chlorphenyl-4-(N,N-methylphenyl)amino-pyrazol,
- 5-Amino-3-(4'-methoxyphenyl)-4-(N,N-methylphenyl)amino-pyrazol,
- 4-Amino-5-methylamino-3-phenyl-pyrazol,
- 4-Amino-5-ethylamino-3-phenyl-pyrazol,
- 4-Amino-5-ethylamino-3-(4'-methylphenyl)-pyrazol,
- 4-Amino-3-phenyl-5-propylamino-pyrazol,
- 4-Amino-5-butylamino-3-phenyl-pyrazol,
- 4-Amino-3-phenyl-5-phenylamino-pyrazol,
- 4-Amino-5-benzylamino-3-phenyl-pyrazol,
- 4-Amino-5-(4'-chlorphenyl)amino-3-phenyl-pyrazol,
- 4-Amino-3-(4'-chlorphenyl)-5-phenylamino-pyrazol,
- 4-Amino-5-(4'-methoxyphenyl)-5-phenylamino-pyrazol,
- 1-(4'-Chlorbenzyl)-4,5-diamino-3-methyl-pyrazol,
- 4,5-Diamino-3-hydroxymethyl-1-isopropyl-pyrazol,
- 4-Amino-1-ethyl-3-methyl-5-methylamino-pyrazol,
- 4-Amino-5-(2'-aminoethyl)amino-1,3-dimethyl-pyrazol und deren Additionssalzen mit einer Säure.

**6.** Zusammensetzung nach Anspruch 5, **dadurch gekennzeichnet, dass** die Diaminopyrazole der Formel (III) ausgewählt sind unter:

- 4,5-Diamino-1,3-dimethyl-pyrazol,
- 4,5-Diamino-3-methyl-1-phenyl-pyrazol,
- 4,5-Diamino-1-methyl-3-phenyl-pyrazol,
- 4-Amino-1,3-dimethyl-5-hydrazino-pyrazol,
- 1-Benzyl-4,5-diamino-3-methyl-pyrazol,
- 4,5-Diamino-3-*t*-butyl-1-methyl-pyrazol,
- 4,5-Diamino-1-*t*-butyl-3-methyl-pyrazol,
- 4,5-Diamino-1-(β-hydroxyethyl)-3-methyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-1-ethyl-3-hydroxymethyl-pyrazol,
- 4,5-Diamino-3-hydroxymethyl-1-methyl-pyrazol,
- 4,5-Diamino-3-hydroxymethyl-1-isopropyl-pyrazol,
- 4,5-Diamino-3-methyl-1-isopropyl-pyrazol,
- 4-Amino-5-(2'-aminoethyl)amino-1,3-dimethyl-pyrazol

und deren Additionssalze mit einer Säure.

**7.** zuaammensetzung nach einem der vorhergehenden Ansprüche, **dadurch gekennzeichnet, dass** das oder die Diaminopyrazol(e) und/oder das oder die entsprechende(n) Additionssalz(e) mit einer Säure 0,0005 bis 12 Gew.-% des Gesamtgewichts der Farbmittelzusammensetzung ausmachen.

**8.** Zusammensetzung nach Anspruch 7, **dadurch gekennzeichnet, dass** das oder die Diaminopyrazol(e) und/oder das oder die entsprechende(n) Additionssalz(e) mit einer Säure 0,005 bis 6 Gew.-% des Gesamtgewichts der Farbmittelzusammensetzung ausmachen.

**9.** Zusammensetzung nach einem der vorhergehenden Ansprüche, **dadurch gekennzeichnet, dass** das oder die halogenierte(n) m-Aminophenol(e) der Formel (I) und/oder das oder die entsprechende(n) Additionssalz(e) mit einer Säure 0,0001 bis 5 Gew.-% des Gesamtgewichts der Farbmittelzusammensetzung ausmachen.

**10.** zuaammensetzung nach Anspruch 9, **dadurch gekennzeichnet, dass** das oder die halogenierte(n) m-Aminophenol (e) der Formel (I) und/oder das oder die entsprechende(n) Additionssalz(e) mit einer Säure 0,005 bis 3 Gew.-% des Gesamtgewichts der Farbmittelzusammensetzung ausmachen.

**11.** Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

**12.** Zusammensetzung nach einem der vorhergehenden Ansprüche, **dadurch gekennzeichnet, dass** das zum Färben geeignete medium (oder der Träger) aus Wasser oder einem Gemisch von Wasser und mindestens einem organischen Lösungsmittel besteht, das unter den niederen $C_{1-4}$-Alkanolen, Glycerid, Glykolen und Glykolethern, aromatischen Alkoholen, analoge Produkten und deren Gemischen ausgewählt ist.

**13.** Zusammensetzung nach einem der vorhergehenden Ansprüche, **dadurch gekennzeichnet, dass** sie einen pH-Wert im Bereich von 3 bis 12 aufweist.

**14.** Zusammensetzung nach einem der vorhergehenden Ansprüche, **dadurch gekennzeichnet, dass** sie als Flüssigkeit, Creme oder Gel oder beliebigen anderen Formen vorliegt, die geeignet sind, Keratinfasern und insbesondere menschliches Haar zu färben.

**15.** Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 14 aufgetragen wird und die Farbe bei einem sauren, neutralen oder alkalischen pH-Wert mit einem Oxidationsmittel entwickelt wird, das kurz vor der Anwendung in die Farbmittelzusammensetzung gegeben wird oder in einer oxidierenden Zusammensetzung vorliegt, die gleichzeitig oder getrennt davon anschließend aufgetragen wird.

**16.** Verfahren nach Anspruch 15, **dadurch gekennzeichnet, dass** das Oxidationsmittel, das in der oxidierenden Zusammensetzung vorliegt, unter Wasserstoffperoxid, Harnstoffperoxid, Alkalimetallbromaten, Salzen von Persäuren, wie Perboraten, Percarbonaten und Persulfaten, und Persäuren ausgewählt ist.

**17.** Vorrichtung mit mehreren Abteilungen oder "Kit" zum Färben mit mehreren Abteilungen, wobei eine erste Abteilung eine Farbmittelzusammensetzung nach einem der Ansprüche 1 bis zu und eine zweite Abteilung eine oxidierende zuaammensetzung enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- DE 3843892
- DE 4234887
- DE 4234886
- DE 4234885
- DE 19543988
- FR 2733749 A
- FR 2586913